(19) Europäisches Patentamt / European Patent Office / Office européen des brevets

(11) **EP 3 542 706 A1**

(12) **EUROPEAN PATENT APPLICATION**
published in accordance with Art. 153(4) EPC

(43) Date of publication:
**25.09.2019 Bulletin 2019/39**

(51) Int Cl.:
*A61B 5/00* (2006.01)   *A61B 5/053* (2006.01)

(21) Application number: **17871546.2**

(22) Date of filing: **15.11.2017**

(86) International application number:
**PCT/KR2017/012918**

(87) International publication number:
**WO 2018/093131 (24.05.2018 Gazette 2018/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.11.2016 KR 20160154137**

(71) Applicant: **Bilab Co., Ltd.**
**Seoul 06779 (KR)**

(72) Inventors:
• **WOO, Eung Je**
**Seongnam-si**
**Gyeonggi-do 13633 (KR)**
• **OH, Tong In**
**Hwaseong-si**
**Gyeonggi-do 18475 (KR)**

(74) Representative: **Cabinet Plasseraud**
**66, rue de la Chaussée d'Antin**
**75440 Paris Cedex 09 (FR)**

(54) **DEVICE FOR MEASURING SLEEP APNEA AND METHOD THEREFOR**

(57)   Disclosed are an apparatus and method for measuring sleep apnea based on images according to impedance data from an upper airway and a thorax of a subject, in which electrical impedance tomography (EIT) using electrodes attached to an upper airway and a thorax is used to generate an image of air distribution inside a lung according to opening and construction of an upper airway to diagnose at least one of among obstructive apnea, central apnea and complex apnea, thereby coming up with a treatment plan.

Fig.1

**Description**

[TECHNICAL FIELD]

The disclosure relates to an apparatus and method for measuring sleep apnea, and more particularly to an apparatus and method for measuring sleep apnea, which diagnoses the apnea using an image based on impedance data from an upper airway and a thorax of a subject and provides information needed for treatment.

[BACKGROUND ART]

Sleep apnea is a serious healthcare problem that causes heart disease, obesity, fatigue, accidents caused by dozing off, etc. and apnea patients have been on the rise worldwide due to increasing life expectancy and increasing overweight people.

To diagnose the sleep apnea, polysomnography (PSG) is generally used, and a medical institution performing the polysomnography installs many instruments for measuring a biometric signal such as a brainwave or the like, an image monitoring device, etc. in a separate laboratory and makes a sleep specialist to operate them.

The sleep apnea includes obstructive apnea, central apnea, and complex apnea.

The obstructive sleep apnea refers to sleep-related breathing disorders showing often wake-up due to disorder in airflow of an upper airway during sleeping and repetitively lowered oxygen concentration in blood, and is defined as a case where breathing efforts are maintained or increased while a condition that a respiratory amplitude is lowered by more than 90% as compared with a base respiratory amplitude for at least 10 seconds is repeated more than five times an hour for adults.

Further, the central sleep apnea refers to apnea symptoms due to many cranial or nervous causes, and is defined as a case where apnea is maintained for more than 10 seconds without breathing efforts.

A patient, who will be subjected to the polysomnography for diagnosing such sleep apnea, is attached with various sensing elements and gets sleep in such a special purpose laboratory, and a polysomnography system analyzes a variety of biometric signals and image data obtained during the sleeping and provides information needed for diagnosis of the sleep apnea to a doctor.

The definition of respiration and apnea may be based on the following bibliography.

(Bibliography: Panossian LA, Avidan AY. Review of sleep disorders. Med Clin North Am 2009;93 (2) :407~25/ Woodson BT, Franco R. Physiology of sleep disordered breathing. Otolaryngol Clin North Am 2007; 40 (4) : 691~711/ Coleman J. Overview of sleep disorders: where does obstructive sleep apnea syndrome fit in Otolaryngol Clin North Am 1999;32(2):187~93/ Redline S, Strohl KP. Recognition and consequences of obstructive sleep apnea hyponea syndrome. Otolaryngol Clin North Am 1999;32(2):303~31/ Thakkar K, Yao M. Diagnostic studies in obstructive sleep apnea. Otolaryngol Clin North Am 2007; 40 (4) : 785~805.)

Here, the polysomnography refers to an examination in which various biometric signals such as brainwaves, electrooculogram (eye movement), jaw electromyogram, electrocardiogram, leg electromyogram, snoring, thoracic-abdominal respiration, saturated oxygen concentration in blood, respiratory airflow, body posture, and the like during the sleeping are simultaneously recorded (synchronized) to provide objective data needed for evaluating a sleep state and diagnosing somnipathy.

However, the polysomnography can be used in comprehensive evaluation with regard to normal respiration or apnea, quality of sleep, etc., but there is a constraint that measurement should be performed with many attached sensing elements in a special-purpose space.

Further, the diagnosis of the obstructive apnea caused by negative pressure in an upper airway may be varied depending on pulmonary functions that differ from person to person, and therefore there is a constraint on analysis of actual influence of apnea on a human body

The obstructive apnea caused by respiratory obstruction and the central apnea caused by nervous system abnormality are different in treatment from each other, and, the upper airway may be obstructed even in the case of central apnea. Therefore, an accurate diagnosis is required.

The diagnosis based on the respiratory will is insufficient in accuracy, and thus there is a need of analyzing both change in air flow from the airway to a lung inside and the respiratory will.

Accordingly, observation of change in flow of air actually introduced into the lung is needed to figure out a more direct cause of sleep apnea and hypopnea, and may be used to come up with a treatment plan for the accurate diagnosis (cause).

[DISCLOSURE]

[TECHNICAL PROBLEM]

The disclosure is to provide an apparatus and method for measuring sleep apnea, in which electrical impedance tomography (EIT) using electrodes attached to an upper airway and a thorax is used to measure opening and obstruction of an upper airway based on an upper airway image and at the same time measure air distribution inside a lung based on a thorax image.

Further, the disclosure is to provide an apparatus and method for measuring sleep apnea, in which air distribution inside a lung according to opening and obstruction of an upper airway is made as an image to diagnose at least one of among obstructive apnea, central apnea and complex apnea, thereby coming up with a treatment plan.

Further, the disclosure is to provide an apparatus and method for measuring sleep apnea, which provides accurate images of an upper airway and a thorax by applying a signal processing technique for separating only a signal component corresponding to a regional change in terms of an algorithm, and suppressing interference between different measured signals.

[TECHNICAL SOLUTION]

An apparatus for measuring the sleep apnea according to an embodiment of the disclosure includes: an upper airway electrode element formed with a plurality of first electrodes for current injection and voltage detection, and attached along face and neck circumferences of a subject to be examined; a thorax electrode element formed with a plurality of second electrodes for current injection and voltage detection, and attached along a chest circumference of a subject to be examined; a controller configured to selectively supply currents to at least one pair of electrodes selected from each of the plurality of first electrodes and the plurality of second electrodes, measure voltage through unselected electrodes, and generate an upper airway image and a thorax image based on upper airway impedance data and thorax impedance data separated from impedance data corresponding to the measured voltage; and an apnea diagnoser configured to diagnose apnea by quantifying the upper airway image and the thorax image.

The apparatus for measuring the sleep apnea according to an embodiment of the disclosure may further include a sensing element configured to sense a biometric signal of the subject.

A method of measuring sleep apnea of a subject by an apparatus for measuring the sleep apnea includes: selectively supplying currents to at least one pair of electrodes selected from each of a plurality of first electrodes attached along face and neck circumferences of the subject and a plurality of second electrodes attached along a chest circumference of the subject; measuring voltage through unselected electrodes among the plurality of first electrodes and the plurality of second electrodes; obtaining impedance data based on the measured voltage; separate upper airway impedance data and thorax impedance data, which are different from each other, by applying a signal separating algorithm to the obtained impedance data; generate an upper airway image and a thorax image based on the upper airway impedance data and the thorax impedance data; and diagnosing apnea by quantifying the upper airway image and the thorax image.

The method of measuring sleep apnea of a subject by the apparatus for measuring the sleep apnea may further include sensing a biometric signal of the subject.

[ADVANTAGEOUS EFFECTS]

According to an embodiment of the disclosure, EIT using electrodes attached to an upper airway and a thorax is used to measure opening and obstruction of an upper airway based on an upper airway image and at the same time measure air distribution inside a lung based on a thorax image.

Further, according to an embodiment of the disclosure, air distribution inside a lung according to opening and obstruction of an upper airway is made as an image to diagnose at least one of among obstructive apnea, central apnea and complex apnea, thereby coming up with a treatment plan.

Further, according to an embodiment of the disclosure, it is possible to provide accurate images of an upper airway and a thorax by applying a signal processing technique for separating only a signal component corresponding to a regional change in terms of an algorithm, and suppressing interference between different measured signals.

[DESCRIPTION OF DRAWINGS]

FIG. 1 is a block diagram of an apparatus for measuring sleep apnea according to an embodiment of the disclosure.

FIG. 2A illustrates the apparatus for measuring the sleep apnea according to an embodiment of the disclosure, and FIG. 2B schematically illustrates an upper airway electrode element and a thorax electrode element.

FIGS. 3A and 3B schematically illustrate a complex electrode employed in the apparatus for measuring the sleep apnea shown in FIG. 2B.

FIGS. 4A to 4D schematically illustrate an electrode belt, and FIG. 4E illustrates an example that the electrode belt is attached to a body of a subject.

FIGS. 5A and 5B illustrate an example of separating impedance data by applying a signal separating algorithm according to an embodiment of the disclosure.

FIGS. 6A and 6B illustrate data and images of an upper airway of a subject measured over time.

FIGS. 7A and 7B illustrate measured data, snoring sound data, and thorax impedance images according to digitized sections from impedance data imaged in a respiratory section and an apnea section in a thorax of a subject.

FIGS. 8A and 8B illustrate change in an upper airway image and a thorax image in an apnea section.

FIG. 9 is a flowchart showing a method of measuring sleep apnea of a subject by the apparatus for measuring the sleep apnea according to an embodiment of the disclosure.

[DETAILED DESCRIPTION OF THE INVENTION]

Below, embodiments of the disclosure will be described with reference to accompanying drawings and content shown the accompanying drawings without limiting the disclosure to the embodiments.

The terminology used herein is for the purpose of describing embodiments only and is not intended to be limiting of the disclosure. As used herein, the singular forms are intended to include the plural forms as well, unless the context clearly indicates otherwise. It will be further understood that the terms "comprises" and/or "comprising" when used herein specify the presence of stated elements, steps, operations, and/or components, but do not preclude the presence or addition of one or more other elements, steps, operations, and/or components.

A certain aspect or design disclosed in "embodiment," "example," "facet," "illustration," etc. used herein should not be construed to be better or more advantageous than other aspects or designs.

Further, terms 'or' are intended to indicate 'inclusive or' rather than 'exclusive or'. In other words, unless mentioned otherwise or the context clearly indicates otherwise, 'x employs a or b' is intended to mean any of the natural inclusive permutations.

Further, articles "a" or "an" as used in the present specification and claims should generally be construed to mean "one or more" unless specified otherwise or clear from context to be directed to a singular form.

Further, it will be understood that, although the terms first, second, etc. may be used herein to describe various elements, these elements should not be limited by these terms. These terms are only used to distinguish one element from another.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined here.

Meanwhile, in the following description, well-known functions or configurations will not be described in detail because they may obscure the gist of the disclosure. Further, the terminologies to be described below are defined in consideration of the functions within the scope of the disclosure and may vary depending on a user's or operator's intention or practice. Accordingly, some definitions are implied based on the content throughout the specification.

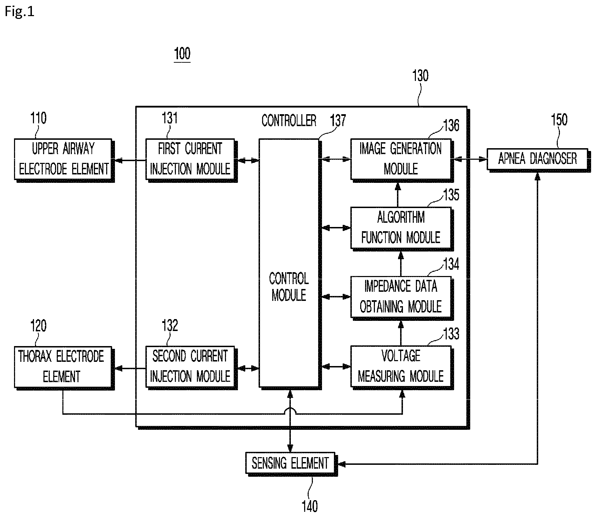

FIG. 1 is a block diagram of an apparatus for measuring sleep apnea according to an embodiment of the disclosure.

Referring to FIG. 1, an apparatus 100 for measuring sleep apnea according to an embodiment of the disclosure diagnoses apnea based on an image of impedance data measured from an upper airway electrode attached to face and neck circumferences of a subject and a thorax electrode attached to a chest circumference.

To this end, the apparatus 100 for measuring the sleep apnea according to an embodiment of the disclosure includes an upper airway electrode element 110, a thorax electrode element 120, a controller 130, and an apnea diagnoser 150.

The upper airway electrode element 110 is provided with a plurality of first electrodes for injecting an electric current and sensing voltage, and attached along the face and neck circumferences of the subject to be examined.

The plurality of first electrodes may be electrical impedance tomography (EIT) electrodes which are attached along the face and neck circumferences of the subject and measure impedance data of the upper airway.

The EIT electrodes may be arrayed on one side of a base plate made of a flexible material and attached to the face and neck circumferences of the subject.

Further, the EIT electrode is used to inject a low current, which cannot be recognized by a user, for example, a high-frequency current of 1mA or below, and measure induced voltage. Current-voltage data measured by the EIT electrode may be used to detect a pattern of an upper airway obstruction and a structural change causing the obstruction through an imaging algorithm.

The thorax electrode element 120 is provided with a plurality of second electrodes for injecting an electric current and detecting voltage, and attached along the chest circumference of the subject to be examined.

The plurality of second electrodes may be EIT electrodes which are attached along the thorax of the subject and measure impedance data of an interior of a lung.

The EIT electrodes may be arrayed on one side of a base plate made of a flexible material and attached to the chest circumference of the subject.

Further, the EIT electrode is used to inject a low current, which cannot be recognized by a user, for example, a high-frequency current of 1mA or below, and measure induced voltage. Current-voltage data measured by the EIT electrode may be used to detect a pattern of a thorax (lung) through the imaging algorithm.

Further, the plurality of first electrodes and the plurality of second electrodes may be at least one of a simple electrode and a complex electrode.

The controller 130 selectively applies an electric current to at least one pair of electrodes selected among the plurality of first electrodes and the plurality of second electrodes, measures voltage through unselected electrodes, and makes an image of upper airway impedance data and thorax impedance data separated from the impedance data based on the measured voltage.

To this end, the controller 130 according to an embodiment of the disclosure may further include a first current injection module 131, a second current injection module 132, a voltage measuring module 133, an impedance data obtaining module 134, an algorithm function module 135, an image generation module 136, and a control module 137.

The first current injection module 131 may be configured to inject the current having a plurality of frequency ranges through at least one pair of electrodes selected among the plurality of first electrodes attached to the face and neck circumferences of the subject.

The second current injection module 132 may be configured to inject the current having a plurality of frequency ranges through at least one pair of electrodes selected among the plurality of second electrodes attached to the thorax of the subject.

According to an embodiment, the first current injection module 131 and the second current injection module 132 may be configured to select a pair of electrodes and a frequency, generate a voltage signal based on the selected frequency, convert the voltage into an electric current, and inject the converted current to each of the face and neck and the thorax of the subject through the selected pair of electrodes.

According to an alternative embodiment, the first current injection module 131 and the second current injection module 132 may be configured to respectively inject two currents converted from two voltage signals different in frequency, or may inject the current by selecting a pair of electrodes among the plurality of second electrodes, which is farthest away from the position of the electrode for injecting the current among the plurality of first electrodes.

In the alternative embodiment, currents different in frequency may be simultaneously injected by selecting one or more pairs of electrodes among the plurality of first electrodes, and currents different in frequency may be simultaneously injected to symmetric pairs of electrodes among the plurality of second electrodes.

In other words, the apparatus 100 for measuring the sleep apnea according to an embodiment of the disclosure may employ a frequency multiplexing method and a spatial multiplexing method in which the relatively distant electrodes are selected as the electrodes for the injection to reduce interference between the signals from the plurality of electrodes.

The voltage signal may be converted into two currents different in phase, and the two currents are converted to have the same amplitude and frequency and respectively injected to the face and neck and the thorax of the subject through the selected pair of electrodes.

The voltage measuring module 133 may be configured to measure voltages induced by the currents injected through the unselected electrodes among the plurality of first electrodes and the unselected electrodes among the plurality of second electrodes.

For example, the voltage measuring module 133 may be configured to remove noises included in detected voltage based on the slope of the measured voltage, and replace a voltage corresponding to a section, a level of which is greater than a preset threshold, by a preset voltage when the slope of the detected voltage is higher than the threshold.

The impedance data obtaining module 134 may be configured to obtain the impedance data based on the measured voltage.

For example, the impedance data obtaining module 134 may be configured to obtain voltage difference signals induced by the injected currents through the plurality of first electrodes and the unselected electrodes among the plurality of second electrodes, and obtain the impedance data according to the positions of the electrodes, the face and neck circumferences and the chest circumference of the subject.

The algorithm function module 135 may be configured to separate the upper airway impedance data and the thorax impedance data by applying a signal separating algorithm to the obtained impedance data, and restore the magnitude of the separated upper airway impedance data and thorax impedance data.

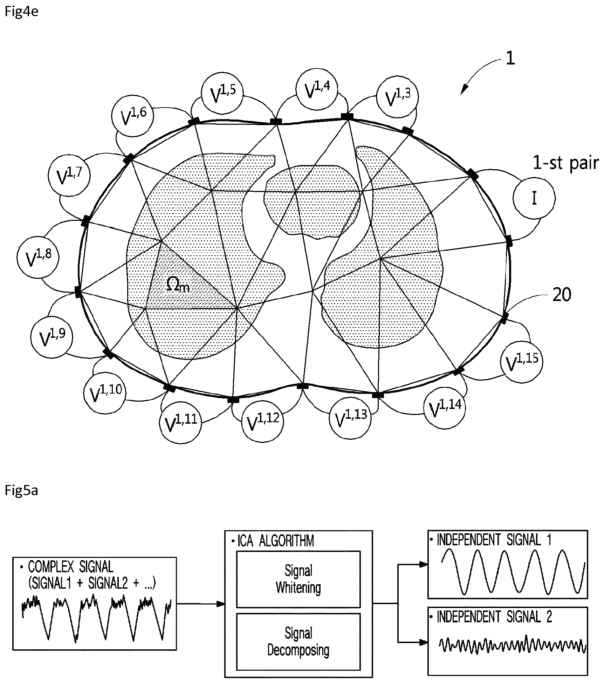

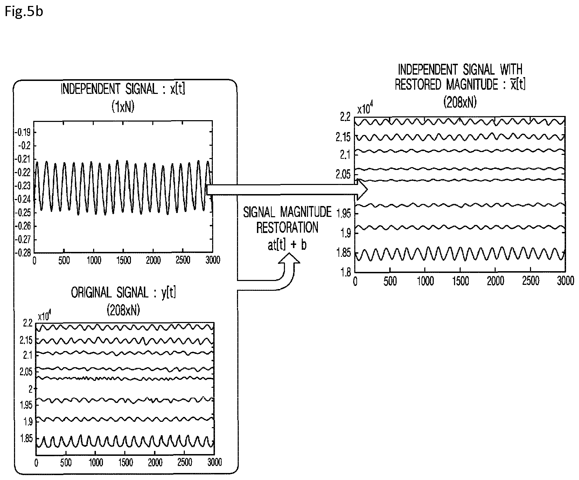

Below, a process of separating the impedance data by applying the signal separating algorithm according to an embodiment of the disclosure will be described with reference to FIGS. 5A and 5B.

FIGS. 5A and 5B illustrate an example of separating impedance data by applying a signal separating algorithm according to an embodiment of the disclosure.

In more detail, FIG. 5A is to describe an example of separating the impedance data by applying the signal separating algorithm, and FIG. 5B is to describe an example of restoring each separated piece of the impedance data to have a magnitude of the original signal.

Referring to FIG. 5A, the apparatus 100 for measuring the sleep apnea according to an embodiment of the disclosure measures voltage in the face and neck circumferences of the subject from the upper airway electrode element 110, and measures voltage in the chest circumference of the subject from the thorax electrode element 120.

Thus, the apparatus 100 for measuring the sleep apnea according to an embodiment of the disclosure receives a complex signal into which different independent signals based on the voltage in the face and neck circumferences of the subject and the voltage in the chest circumference are combined.

For example, there are no impedance changes in an upper airway area because a signal caused by air flow having a constant magnitude change is measured in the thorax during a normal respiratory section, but there is an impedance change in the upper airway area during a section where sleep apnea occurs and therefore a signal caused by air flow inside a lung in the thorax may become weak or disappear.

In this case, the algorithm function module 135 according to an embodiment of the disclosure applies the signal separating algorithm, i.e. an independent component analyses (ICA) algorithm to the combination signal, in which various different independent signals, bio-movement noises and system noises are mixed, and separate independent signals respectively corresponding to the upper airway and the thorax.

In more detail, the algorithm function module 135 according to an embodiment of the disclosure may apply the ICA algorithm for doing signal whitening and signal decomposing to obtain only feature information (i.e. upper airway impedance data and thorax impedance data) from the impedance data which includes the voltage measured in the face and neck circumferences of the subject and the voltage measured in the chest circumference of the subject.

Then, the algorithm function module 135 according to an embodiment of the disclosure may be configured to calculate a signal separation matrix in which independence of signals to be separated by the ICA algorithm is maximized, and use the signal separation matrix to extract different independent signals and remove a noise signal from the combination signal.

Referring to FIG. 5B, the algorithm function module 135 according to an embodiment of the disclosure may be configured to calculate a signal magnitude restoration variable between an independent signal and an original signal by applying a signal magnitude restoration algorithm, in order to restore the magnitude of the independent signals separated by the ICA algorithm to the magnitude of the original signal.

Then, the algorithm function module 135 according to an embodiment of the disclosure may use the calculated restoration variable to restore each independent signal to have the magnitude of the original signal.

In more detail, the algorithm function module 135 according to an embodiment of the disclosure may be configured to obtain the independent signal ($\tilde{x}[t]$), the magnitude of which is restored by applying the following [Expression 1], i.e. the signal magnitude restoration algorithm to the separated independent signal ($x[t]$).

[Expression 1]

$$\begin{bmatrix} y_1 \\ y_2 \\ y_3 \\ \cdot \\ \cdot \\ \cdot \\ \cdot \\ y_N \end{bmatrix} = \begin{bmatrix} x_1 & 1 \\ x_2 & 1 \\ x_3 & 1 \\ & \cdot \\ & \cdot \\ & \cdot \\ x_N & 1 \end{bmatrix} \begin{bmatrix} a \\ b \end{bmatrix} \qquad Y = X\theta$$

$$X^T Y = X^T X \theta$$

$$(X^T X)^{-1} X^T Y = \theta$$

$$\quad Y \qquad\qquad X \qquad\quad \theta$$

where, a and b indicate signal magnitude restoration variables, x indicate an independent signal, and y indicates an original signal.

In other words, the algorithm function module 135 according to an embodiment of the disclosure may be configured to extract the impedance data measured in the upper airway and the thorax of the subject, by a method of applying weight to the voltage data corresponding to the frequency or the position of the pair of electrodes for injecting the current, and separating the weighted data with unique numbers or data sorted according to the measured frequency or position.

Referring back to FIG. 1, the image generation module 136 in the apparatus 100 for measuring the sleep apnea according to an embodiment of the disclosure may be configured to generate the upper airway image and the thorax image based on the restored upper airway impedance data and thorax impedance data.

For example, the image generation module 136 may be configured to generate the upper airway image and the thorax image by restoring conductivity and permittivity images of the subject with the measured upper airway impedance data and thorax impedance data.

Specifically, the image generation module 136 may be configured to have measurement protocols, which the plurality of first electrodes and the plurality of second electrodes have, to make images the upper airway and the thorax of the subject, thereby controlling sensitivity and resolutions according to the positions of the upper airway electrode element 110 and the thorax electrode element 120. With measurement values using such a plurality of measurement protocols or combination thereof, and a sensitivity matrix improved in a three-dimensional (3D) modeling image generated through the upper airway electrode element 110 and the thorax electrode element 120, conductivity and permittivity images corresponding to the upper airway of the subject and the interior of the thorax, i.e. the upper airway image and the thorax image may be generated as the impedance images.

The control module 137 may be configured to control selection of at least one pair of electrodes from each of the plurality of first electrodes and the plurality of second electrodes, control selection of the unselected electrodes, and control sensing of a biometric signal of the subject.

The control module 137 may be configured to control the first current injection module 131 and the second current injection module 132 to measure the impedance data with regard to at least one of the upper airway and the thorax of the subject, and control the sensing element 140 to sense a biometric signal with regard to a measurement target part of the subject.

Further, the control module 137 may control at least one module among the voltage measuring module 133, the impedance data obtaining module 134, the algorithm function module 135, and the image generation module 136 so as to measure vertical and horizontal impedance with regard to at least one of the upper airway and the thorax of the subject.

Further, the control module 137 may control a user interface (not shown) to display and provide information based on diagnosis of sleep apnea to a user, or control a communicator (not shown) to transmit the information to the outside.

In obtaining the upper airway image and the thorax image of the subject with the apparatus 100 for measuring the sleep apnea according to an embodiment of the disclosure, the EIT method is as follows.

The controller 130 according to an embodiment of the disclosure selects channel and sinusoidal frequencies in response to a command, and selects a pair of electrodes in the upper airway electrode element 110 and the thorax electrode element 120 corresponding to the selected channel. The selected pair of electrodes are used for injecting the current to the upper airway corresponding to the face and neck and the thorax of the subject, and the unselected electrodes are used for measuring the voltage on the subject.

When the channel and sinusoidal frequencies are selected, the controller 130 outputs a control signal for controlling a field programmable gate array (FPGA, not shown). The control signal may include information about the selected frequencies.

The FPGA is configured to receive and store the control signal, and generate a sinusoidal voltage signal based on the received control signal. In particular, the FPGA is configured to generate a voltage signal based on frequency information included in the control signal, and transmits the generated voltage signal to two 16bit D/A converters (not shown). In this case, the FPGA controls a 8bit D/A converter (not shown) to adjust the amplitude of the voltage signal transmitted to the 16bit D/A converter. Then, the voltage signals output to the two 16bit D/A converters are converted into electric currents by the voltage-the current converters (not shown), and the two currents are transmitted to a corrector (not shown). The corrector (not shown) adjusts the two currents to have the same amplitude and frequency. Here, there is a phase difference of 180° between the two currents.

The control module 137 of the controller 130 according to an embodiment of the disclosure may set the first current injection module 131 and the second current injection module 132 to be different in frequency and phase difference so as to simultaneously measure the upper airway impedance and the thorax impedance, and may use control the frequency multiplexing and spatial multiplexing methods to minimize the interference by controlling the positions of the electrodes for simultaneous injection.

In addition, the control module 137 of the controller 130 controls the first current injection module 131 and the second current injection module 132 to respectively transmit two currents passed through the corrector to the pair of electrodes selected in the upper airway electrode element 110 and the thorax electrode element 120.

The currents injected to the face and neck circumferences of the subject and the chest circumference causes the surface thereof to induce voltages different in level according to resistivity or conductivity of internal tissues. When the electrodes unselected in the upper airway electrode element 110 and the thorax electrode element 120 senses the voltages on the surface of the face and neck circumferences and the surface of the chest circumference of the subject, the voltage measuring module 133 receives the voltages on the surfaces corresponding to the unselected electrodes.

Then, the voltage measuring module 133 determines whether the surface voltage data includes noises based on the slope of the sensed surface voltage data, and then replaces the corresponding voltage data by another voltage level when the surface voltage data includes the noises. Further, the control module 137 adjusts a gain of the voltage amplifier (not shown) according to the maximum level of the voltage data. For example, the control module 137 does not adjust the gain of the voltage amplifier when the maximum level of the voltage data reaches 90% of the maximum output of the A/D converter (not shown), but increases the gain of the voltage amplifier when the maximum level of the voltage data does not reach 90% of the maximum output of the A/D converter.

When the noises are removed from the voltage data and the gain of the voltage amplifier is adjusted, the voltage measuring module 133 amplifies the voltage data according to the adjusted gain and the A/D converter converts the voltage data into a digital value.

Then, the impedance data obtaining module 134 processes the voltage data in consideration of gain information according to channels, based on the channel information and the gain information. When the detected voltage data different in gain is directly used, it is difficult to accurately represent electric characteristics inside the upper airway and the thorax of the subject. Therefore, the corresponding voltage level has to be decreased or increased according to the gain. For example, when a gain value is greater than a reference gain value, the corresponding voltage level is decreased and multiplied by a ratio of the gain value to the reference gain value.

Thus, the impedance data obtaining module 134 may be configured to process the voltage data in consideration of the gain information according to the channels, and then obtain the impedance data based on the voltage data.

Then, the algorithm function module 135 separates the upper airway impedance data and the thorax impedance data from the impedance data, and the image generation module 136 generates an interior image of the upper airway and the thorax of the subject based on the upper airway impedance data and the thorax impedance data. Alternatively, various methods of generating an image of an inside of a measurement target (an upper airway or a thorax) based on the voltage data on the surface of the upper airway and the thorax of the subject.

Further, the image generation module 136 may use an optical imaging device and a length measuring device to obtain outer appearance information based on markers positioned at the electrodes so as to form a 3D restoration model according to the shapes of the lower-jaw and chest of the subject, and thereby providing a restoration algorithm.

Here, the restoration algorithm refers to an algorithm for restoring a 3D image, and employs a generally used algorithm.

The apparatus 100 for measuring the sleep apnea according to an embodiment of the disclosure may further include the sensing element 140 for sensing a biometric signal of the subject.

For example, the sensing element 140 may include a plurality of sensors, which are fiber-based sensors to perform a function of sensing a biometric signal of a user who is sleeping.

According to an embodiment, the sensing element 140 may include at least one of an oxygen-saturation-in-blood sensor for sensing a signal of oxygen saturation in arterial blood (saturation of peripheral oxygen, SpO2) at parts targeted to be examined while the subject is sleeping, a sound sensor for sensing sound caused by biological activities of the subject, a pose sensor for sensing movement of the subject, and an electrocardiogram sensor for sensing an electrocardiogram at parts targeted to be examined.

Here, the oxygen-saturation-in-blood sensor may be attached to the parts of the subject targeted to be examined and sense oxygen saturation in blood (Sp02), which is present in hemoglobin among many components of blood.

According to an embodiment, the oxygen-saturation-in-blood sensor may be configured to sense a signal about photoplethysmography (PPG) of a body of the subject based on reflected or transmitted light, and sense the oxygen saturation in blood based on the sensed signal about the PPG.

Further, the sound sensor may be configured to sense at least one sound among breathing, snoring, crying, and sleep-talking. According to an embodiment, the sound sensor may be attached to the parts of the subject targeted to be examined or may be given in a contactless form as being spaced at a predetermined distance from the subject while the subject is sleeping.

Further, the posture sensor may be provided as at least one of a gyro sensor and an acceleration sensor, and attached to the parts of the subject targeted to be examined, thereby sensing the posture according to the movement of the subject.

For example, the posture sensor may use the acceleration sensor to sense change in a sleeping posture of the subject, for example, sense change in the sleeping posture as the subject lies down while sleeping or sits down or stands up while waking.

Further, the posture sensor may be positioned on the thorax and abdomen of the subject, and configured to sense subtle change in the parts due to breathing, thereby determining the respiratory will based on the subtle change.

The electrocardiogram sensor may be in contact with the parts of the subject targeted to be examined, and configured to sense an electrocardiogram (ECG).

Here, the ECG shows a waveform based on a vector sum corresponding to action potential generated by a special excitatory & conductive system of a heart. In other words, the ECG may refer to a vector sum signal of action potential generated in parts of a heart, i.e. a sinoatrial (SA) node, an atrioventricular (AV) node, a His bundle, a bundle branch, Purkinje fibers, etc. is measured by the electrodes attached to the exterior of the body.

According to an alternative embodiment, the sensing element 140 may sense at least one of electroencephalogram (EEG), electromyogram (EMG), electrooculogram (EOG), seismocardiogram (SCG) of the thorax, and ballistocardiogram (BCG) of the subject.

In the apparatus 100 for measuring the sleep apnea according to an embodiment of the disclosure, the sensing element 140 according to an embodiment may further include a sleeping environment sensor for sensing sleeping environments, and the sleeping environment sensor may be placed at a predetermined distance from the subject and sense at least one of noise, light, vibration, temperature, and humidity in a sleeping space.

Referring to FIG. 1, the apnea diagnoser 150 of the apparatus 100 for measuring the sleep apnea according to an embodiment of the disclosure diagnoses the apnea by quantifying the upper airway image and the thorax image.

The apnea diagnoser 150 may be configured to quantify at least one of the opening and obstruction change, degree and pattern of the upper airway over time based on the upper airway image, and quantify at least one of air distribution change, degree and pattern inside a lung over time based on the thorax image.

Thus, the apnea diagnoser 150 may be configured to diagnose at least one of the obstructive apnea, the central apnea and the complex apnea based on results from quantification of the upper airway image and the thorax image and the biometric signal sensed by the sensing element 140.

For example, the apnea diagnoser 150 may use the quantified upper airway and thorax images, and the sensed biometric signal to detect the sleeping state of the subject.

The apnea diagnoser 150 may detect the respiration or apnea of the subject who is sleeping, based on the quantified upper airway and thorax images, and morphology information such as the ECG, BCG and SCG from the sensed biometric signal, the movement of the thorax and abdomen, etc.

According to an embodiment, the apnea diagnoser 150 may obtain peak intervals in the biometric signal of the ECG based heart rate variability caused by breathing called respiratory sinus arrhythmia, and apply interpolation to the intervals, thereby detecting a respiratory signal. Therefore, the apnea diagnoser 150 can detect the apnea state from the respiratory signal.

In other words, the apnea diagnoser 150 may detect the respiration or apnea of the subject who is sleeping, based on the biometric signal of the ECG and the upper airway and thorax images corresponding to the upper airway and the thorax of the subject.

Further, when the apnea diagnoser 150 detects the sleep state of the apnea, the sleep state of the obstructive apnea or the central apnea may be detected based on the biometric signal and comparison between the upper airway image showing opening or obstruction of the upper airway and the thorax image corresponding to the air distribution inside the lung.

For example, the apnea diagnoser 150 may use the upper airway and thorax images and the biometric signal to detect the sleep state with the obstructive apnea showing often wake-up due to disorder in airflow of the upper airway of the subject and repetitively lowered oxygen concentration in blood, or the central apnea showing the apnea for more than 10 seconds or no breathing efforts.

Further, the apnea diagnoser 150 may compare the upper airway image and the thorax image in a section (time) where the apnea occurs, and thus diagnose at least one apnea symptom among the obstructive apnea where the upper airway is obstructed by an external cause, the central apnea where a command for breathing is not signaled because of abnormality in a cerebrum or brain stem and therefore respiratory movement is stopped, and the complex apnea where both the obstructive apnea and the central apnea are shown.

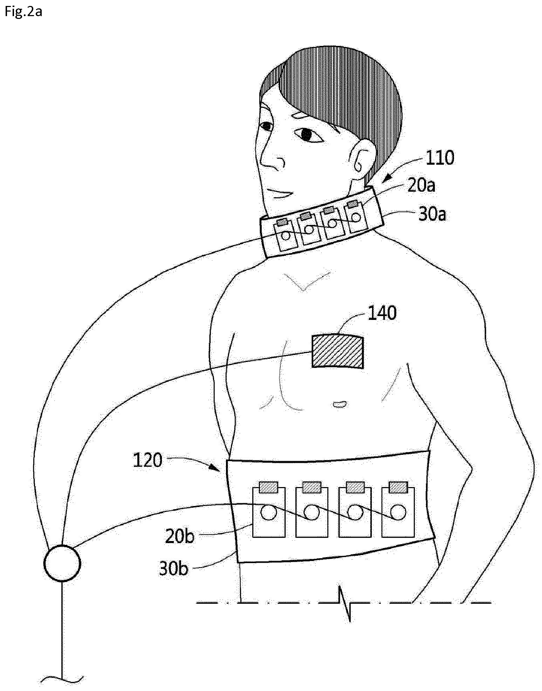

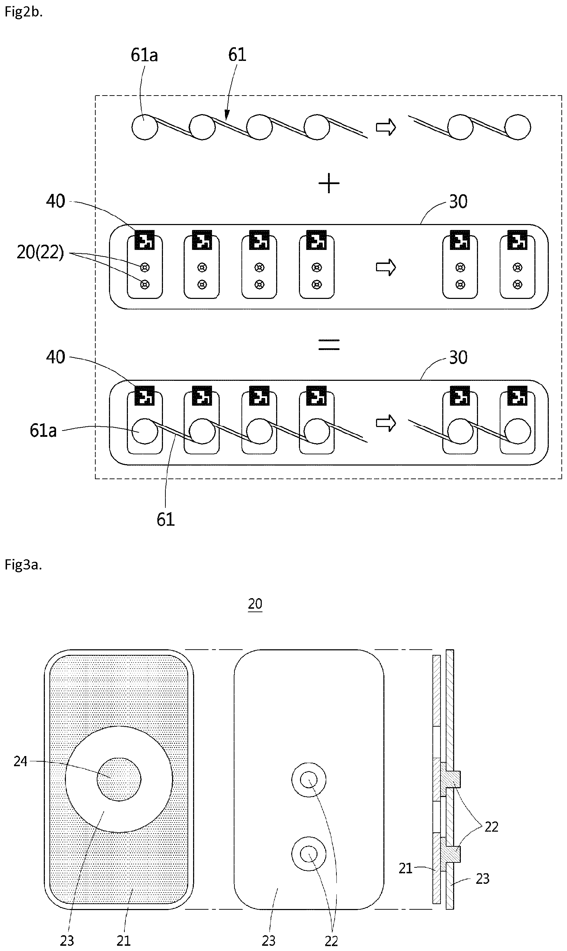

FIG. 2A illustrates the apparatus for measuring the sleep apnea according to an embodiment of the disclosure, and FIG. 2B schematically illustrates an upper airway electrode element and a thorax electrode element.

Referring to FIG. 2A, the apparatus for measuring the sleep apnea according to an embodiment of the disclosure includes the upper airway electrode element 110 and the thorax electrode element 120 which can be respectively attached to the upper airway of the subject and the thorax of the user, and the sensing element 140 attached to parts of the subject targeted to be examined.

The upper airway electrode element 110 attached to the face and neck circumferences of the subject is formed at regular intervals on the base plate 30a and attached to a facial skin around the upper airway area, thereby measuring the impedance data related to the opening and obstruction of the upper airway.

Here, the base plate 30a formed with the upper airway electrode element 110 according to an embodiment of the disclosure may be provided in the form of at least one among a mask-type plate, a belt-type plate, an electrode belt, and a cable belt, but not limited thereto.

Further, the upper airway electrode element 110 may be formed in a belt- or mask-type protective gear formed to surround a plurality of first electrodes 20a, thereby protecting the plurality of first electrodes 20a attached to the facial skin around the upper airway area.

Such a protective gear serves to not only relieve a degree of pressure a user feels while getting natural sleep but also maintain contact strong enough to measure the induced voltage, and gives much less pressure than the pressure of the existing ultrasonic probe. Therefore, the upper airway electrode element 110 with such a structure makes it easy to measure for a long time whether a user's upper airway is obstructed during natural sleep.

According to an embodiment, the plurality of first electrodes 20a may include conductive fiber electrodes manufactured based on silver (Ag)-plated elastic fiber or polymer nanofiber web (PVDF), but be not limited thereto. Alternatively, the electrodes may be made of various materials having less reaction with skin even against long-time measurement.

The thorax electrode element 120 to be attached to the chest circumference of the subject is formed at regular intervals on a base plate 30b and attached to the thorax of the subject, thereby measuring impedance from the shape of the lung corresponding to the respiration while the subject is sleeping.

According to an embodiment, the base plate 30b formed with the thorax electrode element 120 according to an embodiment of the disclosure is not necessarily limited to the shape of the belt-type array electrode. Alternatively, the base plate 30b may include the thorax electrode element 120 having an array of at least one pattern or structure among a vest type, a belt type, and a patch type, by taking contact strength for enhancing a data measurement level into account while minimizing pressure that the subject feels during the natural sleep.

In FIG. 2A, a plurality of electrodes 20 in the upper airway electrode element 110 and the thorax electrode element 120 according to an embodiment of the disclosure may be formed on a base plate 30 at regular intervals or may be arranged with various arrays and structures on the parts (the upper airway and the thorax) targeted to be examined according to the features and purposes. Further, the base plate 30 may have a certain length and area in order to measure impedance while surrounding the parts targeted to be examined, such as the upper airway and the thorax or the abdomen of the subject, but there are no limits to the length and the area. Alternatively, the length and the area may be varied depending on embodiments.

Further, the upper airway electrode element 110 and the thorax electrode element 120 according to an embodiment of the disclosure may effectively measure distribution of an electric field around the surfaces of the upper airway or the thorax based on change in an electrode array structure and a measurement structure in such a manner that the plurality of electrodes 20 are arranged in the form of a 2D or 3D array as attached to the upper airway or the thorax of the subject.

According to an embodiment, the upper airway electrode element 110 and the thorax electrode element 120 to be formed on the base plate 30 are arranged in the 3D array and configured to measure impedance corresponding to each layer, thereby leading to a more accurate and effective diagnosis than that of a conventional method of providing only a 2D cross-section image at a certain position.

In the apparatus for measuring the sleep apnea according to an embodiment of the disclosure, the sensing element 140 attached to parts of the subject targeted to be examined is in contact with the parts of the subject targeted to be examined and senses a biometric signal.

The sensing element 140 may contact any part of the subject targeted to be examined, and therefore the position and number of parts targeted to be in contact are not limited to those shown in FIG. 2A.

According to an embodiment, the sensing element 140 may include at least one of the sound sensor, the posture sensor and the ECG sensor, and may be a fiber-based sensor to be attached to the body of the subject. Further, the sensing element 140 may refer to a plurality of sensors.

According to an embodiment, the plurality of sensors may be attached to different parts on the body of the subject, and the sensing element 140 may be a common name of the plurality of sensors.

Further, the sensing element of the apparatus for measuring the sleep apnea according to an embodiment of the disclosure may sense an oxygen-saturation-in-blood (Sp02) signal through an optic sensor, which senses the amount of red light transmitting blood flowing in a peripheral blood vessel at a fingertip or a tip toe, so as to measure the oxygen saturation in blood of the subject.

For example, the sensing element may be provided in the form of a measurement terminal put on the fingertip, and may be actualized by an optic module that includes a red light emitting diode (LED) of 660nm and an infrared LED of 940nm as a light emitter, and a photodiode and a phototransistor as a light receiver.

Referring to FIG. 2B, the upper airway electrode element 110 and the thorax electrode element 120 according to an embodiment of the disclosure include a plurality of electrodes 20, and are mountable along the face and neck circumferences or the chest circumference of the subject to be examined. To this end, the upper airway electrode element 110 and the thorax electrode element 120 include the base plate 30 (hereinafter, referred to as an electrode belt) provided with the plurality of electrodes 20.

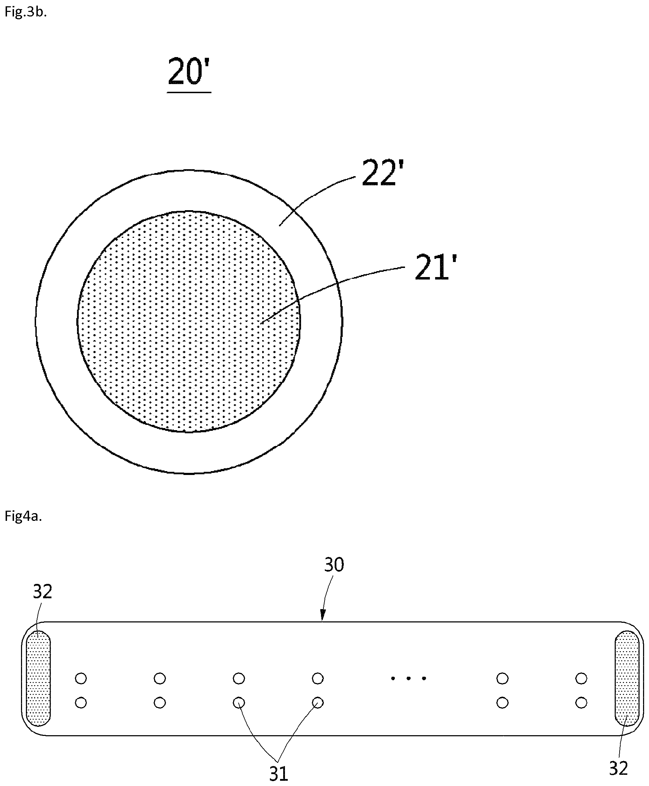

Below, the electrode belt with the plurality of electrodes 20 will be described in detail with reference to FIGS. 3A and 3B.

FIGS. 3A and 3B schematically illustrate a complex electrode employed in the apparatus for measuring the sleep apnea shown in FIG. 2B.

Referring back to FIG. 2B, a cable belt 61 is connected to a connector 22 exposed through an electrode installation hole 31 in which the plurality of electrodes 20 (hereinafter, referred to as the complex electrode) is installed. In this case, the cable belt 61 may include a plurality of connection cable terminals 61a for injecting the currents, corresponding to the complex electrodes 20.

Thus, the voltage measuring module 133 in the apparatus 100 for measuring the sleep apnea according to an embodiment of the disclosure measures the voltage induced by the current injected to the complex electrode 20 through the cable belt 61. Specifically, the currents having multiple frequencies are generated through the cable belt 61 and supplied to the plurality of electrodes 20 of the electrode belt 30 put on the subject while being subjected to amplitude and phase control. In this case, the currents having multiple frequencies are injected through a first electrode 21 of the complex electrode 20, and a voltage difference signal induced by the injected current is obtained through a second electrode 24 of the complex electrode 20.

Referring to FIG. 3A, the complex electrode 20 includes the first electrode 21 made of a conductive material for injecting a current, the second electrode 24 made of a conductive material for measuring a voltage, and a connector 22 shaped like a button to be connected to the cable belt 61. The first electrode 21 injects the current through a relatively large area as compared with the second electrode 24, and the second electrode 24 measures the voltage through a relatively small area as compared with the first electrode 21 and forms a pair with the second electrode 24 of the repetitive complex electrodes 20 on the cable belt 61.

In this case, the first electrode 21 is shaped like a flat plate, and the connector 22 shaped like a button is provided in the form of a pair of projections which protrude as being connected to the first electrode 21 and the second electrode 24. The first and second electrodes 21 and 24 of each of the plurality of complex electrodes 20 are installed with a nonconductor 23 made of a nonconductive material therebetween in the electrode belt 30.

The foregoing example illustrates that the plurality of electrodes 20 includes the complex electrode, but the plurality of electrodes 20 is not limited to this example. As an alternative example, the simple electrode 20' as shown in FIG. 3B is also possible. In a case of the simple electrode 20', the injection of the current or the measurement of the voltage is performed in a single conductive electrode 21' which is supported on a nonconductor 22'.

Further, the electrode 20 or 20' may be made of a flexible conductive fiber or a conductive polymeric material, and may be given in the form of a dry-type electrode.

FIGS. 4A to 4D schematically illustrate an electrode belt, and FIG. 4E illustrates an example that the electrode belt is attached to a body of a subject.

Referring to FIG. 4A, the electrode belt 30 is made of an elastic material such as fiber, silicon and the like polymeric compound, and the number of provided electrode installation holes 31 and the number of installed complex electrodes 20 may be variable. Meanwhile, the electrode belt 30 is provided with a pair of fasteners 32 at opposite ends thereof, which are fastened to each other and maintain the electrode belt 30 as wound around the body of the subject.

This embodiment of the disclosure illustrates that the electrode belt 30 is wound around parts of the subject targeted to be examined, i.e. the face and neck circumferences or the chest circumference, and fastened by Velcro type fasteners 32 provided at the opposite ends thereof. Alternatively, without limitations, one of various fasteners such as hook types, etc. may be employed as the fasteners 32.

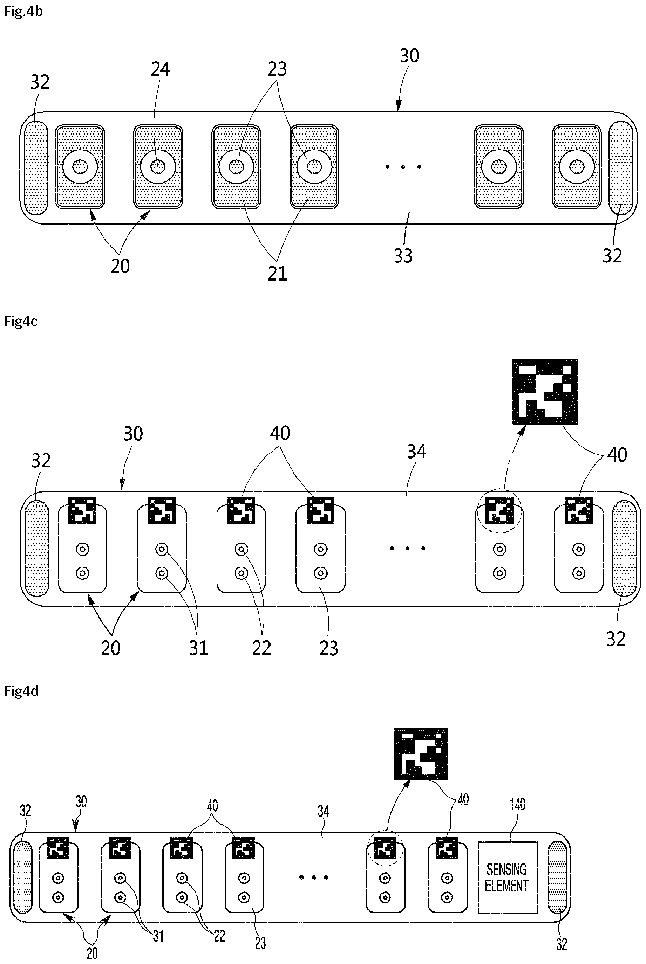

The electrode belt 30 includes a contact surface 33 to be in contact with the subject while having the plurality of complex electrodes 20 as shown in FIG. 4B, and an exposure surface 34 to be exposed to the controller 130 according to an embodiment of the disclosure while being opposite to the contact surface 33 as shown in FIG. 4C. In this case, the first electrode 21 and the second electrode 24 of the complex electrode 20 are exposed on the contact surface 33 of the electrode belt 30, and the connector 22 connected to the cable belt 61 is exposed through the electrode installation hole 31 on the exposure surface 34. In addition, the exposure surface 34 of the electrode belt 30 includes a plurality of indicators 40, i.e. markers respectively corresponding to the plurality of complex electrodes 20 and having a plurality of colors and patterns corresponding to information of each of the plurality of complex electrodes 20.

According to an embodiment, the indicators 40, i.e. the markers may be different in shape according to the complex electrodes 20, and include corresponding channel numbers or pieces of data information different from one another, so that the position of the electrode can be identified as the indicator 40 is recognized (sensed).

Referring to FIG. 4D, the electrode belt 30 may further include the sensing element 140 to be attached to the parts of the subject targeted to be examined, and thus be, together with the sensing element 140, attached to the skin of the subject.

The sensing element 140 will not be described because the features thereof are described above with reference to FIGS. 1 and 2A.

Referring to FIG. 4E, the complex electrodes 20 included in the electrode belt are arranged in the form of the 3D array along the body circumference of the subject 1 to be examined, and it is thus possible to obtain a 3D image at a certain position (the thorax or the upper airway) because impedance corresponding to each layer is measurable by injecting the currents through the selected pair of electrodes and measuring the voltage induced by the injected currents.

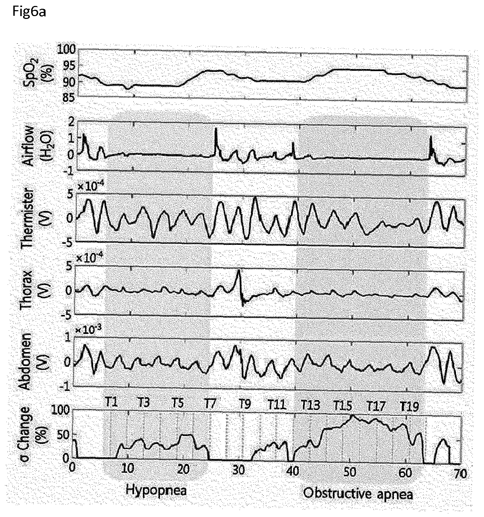

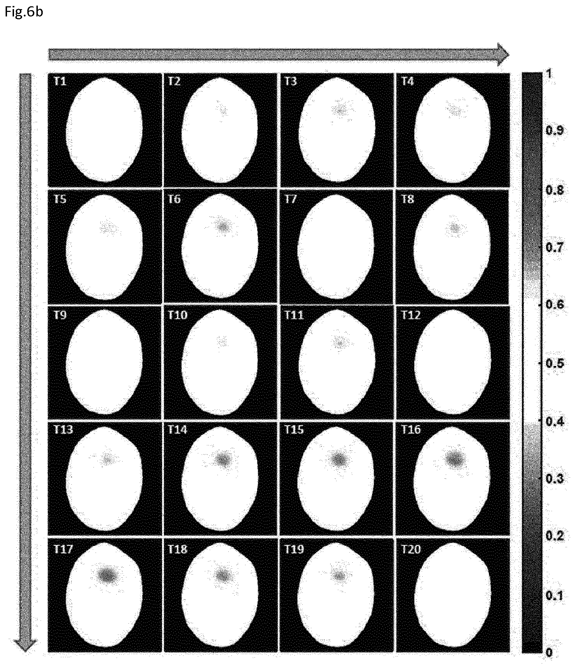

FIGS. 6A and 6B illustrate data and images of an upper airway of a subject measured over time.

FIG. 6A illustrates measured data of a biometric signal sensed at parts of the subject targeted to be examined by the apparatus for measuring the sleep apnea according to an embodiment of the disclosure.

In more detail, the apparatus for measuring the sleep apnea according to an embodiment of the disclosure may collect data measured over time with respect to each of the oxygen saturation in blood(Sp02), the air flow, the thermister, the thorax, and the abdomen of the subject.

Thus, the apparatus for measuring the sleep apnea according to an embodiment of the disclosure may sense a hypopnea section or an obstructive apnea section, and measure time T1, T2, ···, T19 corresponding to the sensed sections.

Referring to FIG. 6B, the apparatus for measuring the sleep apnea according to an embodiment of the disclosure may obtain the upper airway image over the periods of time T1, T2, ···, T19, and thus identify whether the upper airway is opened or obstructed during the hypopnea or the apnea.

Referring to FIGS. 6A and 6B, it is possible to more clearly, accurately, and quantitatively identify whether the upper airway is opened or obstructed in the hypopnea section and the apnea section, based on comparison between the upper airway image corresponding to the hypopnea section T1 to T7 and the upper airway image corresponding to the apnea section T12 to T20.

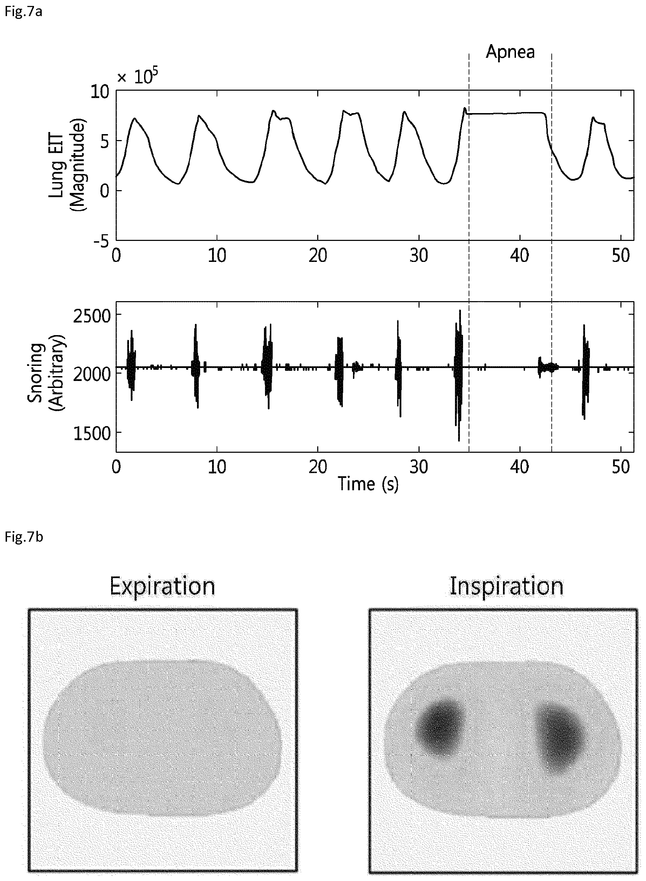

FIGS. 7A and 7B illustrate measured data, snoring sound data, and thorax impedance images according to digitized sections from impedance data imaged in a respiratory section and an apnea section in a thorax of a subject.

FIG. 7A illustrates a snoring signal sensed from the sensing element and measured data of lung EIT based on voltage sensed in the thorax of the subject by the apparatus for measuring the sleep apnea according to an embodiment of the disclosure.

In more detail, the apparatus for measuring the sleep apnea according to an embodiment of the disclosure can obtain the lung EIT measured data based on the air distribution inside the lung, and an apnea section based on the measured snoring signal.

FIG. 7B illustrates a thorax image from the sensed apnea section.

Referring to FIG. 7B, the apparatus for measuring the sleep apnea according to an embodiment of the disclosure identify expiration and inspiration corresponding to air distribution inside the lung, based on the thorax image.

Therefore, a base amount of air in the lung and a changed amount during the apnea and hypopnea may be made as an image based on the thorax impedance image.

Therefore, referring to FIG. 7A and 7B, the apparatus for measuring the sleep apnea according to an embodiment of the disclosure may determine the apnea of the subject based on the voltage measured from the thorax electrode element, the sensed biometric signal (e.g. snoring signal), and the thorax image.

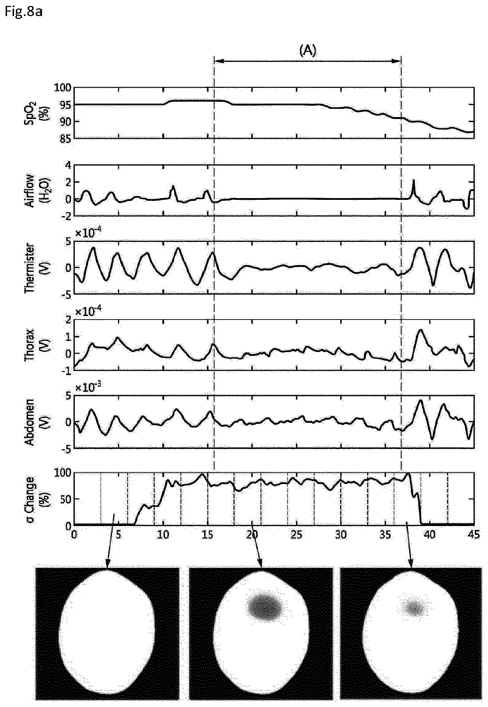

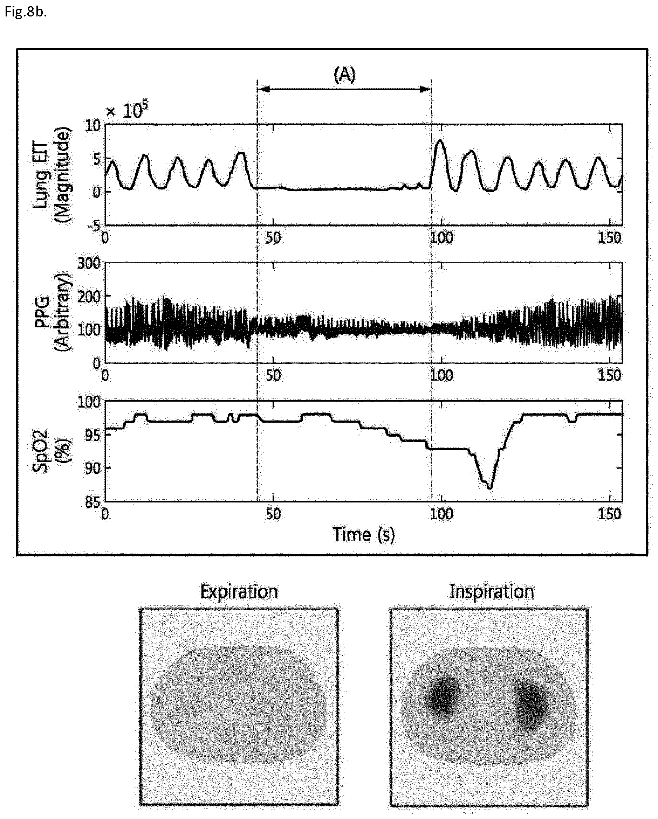

FIGS. 8A and 8B illustrate change in an upper airway image and a thorax image in an apnea section.

FIG. 8A illustrates opening and obstruction change in the upper airway according to the upper airway image measured in the apnea section (A), and FIG. 8B illustrates change in the air distribution inside the lung according to the thorax image measured in the apnea section (A).

In more detail, the upper airway image in the sections other than the apnea section (A) of FIG. 8A shows that the upper airway is open (see the leftmost upper airway image. No changes are shown in the image because the upper airway is always open in a normal state). Further, the upper airway image in the apnea section (A) shows that the upper airway is obstructed (see the middle upper airway image. Change in the impedance image is shown because the upper airway is obstructed and internal tissues are changed), and the upper airway image immediately before the end of the apnea section (A) shows that the upper airway is in the middle of being opened (see the rightmost upper airway image).

Referring to FIG. 8B, the apparatus for measuring the sleep apnea according to an embodiment of the disclosure detects the apnea section (A) from the impedance data based on the voltage measured at the thorax electrode element, the PPG, and Sp02, and identifies change in air between expiration and inspiration according to the air distribution inside the lung based on the thorax image in the detected apnea section (A).

Thus, the apparatus for measuring the sleep apnea according to an embodiment of the disclosure identifies the air distribution inside the lung and whether the upper airway is opened or obstructed in the apnea section (A) and diagnoses at least one among the obstructive apnea, the central apnea and the complex apnea.

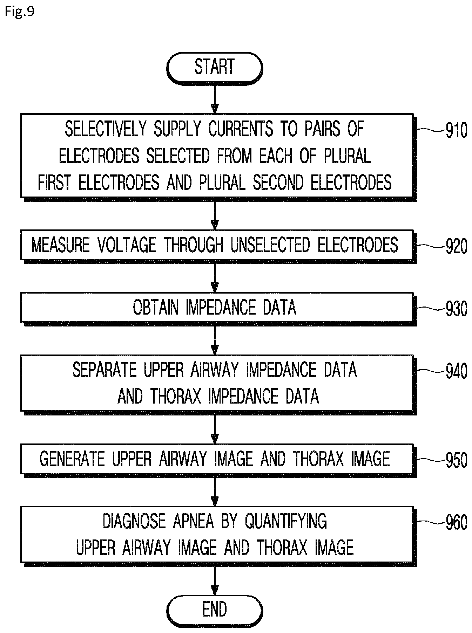

FIG. 9 is a flowchart showing a method of measuring sleep apnea of a subject by the apparatus for measuring the sleep apnea according to an embodiment of the disclosure.

Referring to FIG. 9, at operation 910, electric current are selectively supplied to at least one pair of electrodes selected among the plurality of first electrodes attached along the face and neck circumferences of the subject and among the plurality of second electrodes attached along the chest circumference of the subject.

The operation 910 may include operation at which the pair of electrodes is selected among the plurality of first electrodes, the pair of electrodes is selected among the plurality of second electrodes, frequencies are selected, voltage signals are generated based on different selected frequencies and converted into currents, and the converted currents are respectively injected to the face and neck and the thorax of the subject through the selected pairs of electrodes.

At operation 920, voltages are measured through the unselected electrodes among the plurality of first electrodes and the plurality of second electrodes.

At operation 930, impedance data is obtained based on the measured voltage.

At operation 940, the obtained impedance data is subjected to the signal separating algorithm, and thus the upper airway impedance data and the thorax impedance data different from each other are separated from a biometric noise signal.

The operation 940 may include operation at which the ICA algorithm, i.e. the signal separating algorithm is applied to the obtained impedance data so that the upper airway impedance data and the thorax impedance data different from each other are separated, and the signal magnitude restoration algorithm is applied to each separated impedance data so that the original signal can be restored.

At operation 950, the upper airway image and the thorax image are made based on the upper airway impedance data and the thorax impedance data.

The operation 950 may include operation at which the upper airway image and the thorax image are made from the restored upper airway and thorax impedance data.

According to an embodiment of the disclosure, the method of measuring the sleep apnea may further include operation (not shown) of sensing the biometric signal of the subject.

At operation 960, the upper airway image and the thorax image are quantified to thereby diagnose the apnea.

The operation 960 may include operation at which at least one among the obstructive apnea, the central apnea and the complex apnea is diagnosed based on the sensed biometric signal and the results from the quantification of the upper airway and thorax images.

The method according to the embodiments may be actualized in the form of program instructions to be implemented through various computing means, and recorded in a computer readable medium. The computer readable medium may include a program instruction, a data file, a data structure solely or in combination. The program instructions recorded in the medium may be specially designed and configured for the embodiments or may be available as publicly known to a person having an ordinary skill in the art of computer software. The computer readable medium may for example include a hard disk, a floppy disk, a magnetic tape and the like magnetic media; a CD-ROM, a DVD and the like optical media; a floptical disk and the like magneto-optical media; and a ROM, a RAM, a flash memory and the like hardware device specially configured to store and implement a program instruction. The program instruction may for example include not only a machine code produced by a compiler, but also a high-level language to be implemented by a computer through an interpreter or the like. The hardware device may be configured to operate as one or more software modules to carry out the operations of the embodiment, and vice versa.

Although few embodiments are described with restricted examples and drawings, various modifications and changes can be made in the embodiments by a person having an ordinary skill in the art. For example, suitable results may be achieved even if the described techniques are performed in a different order, and/or if components in a described system, architecture, device, or circuit are combined in a different manner and/or replaced or supplemented by other components or their equivalents.

Therefore, other realizations, other embodiments and equivalents to claims may also belong to the scope of appended claims.

**Claims**

1.  An apparatus for measuring sleep apnea, comprising:

    an upper airway electrode element formed with a plurality of first electrodes for current injection and voltage detection, and attached along face and neck circumferences of a subject to be examined;
    a thorax electrode element formed with a plurality of second electrodes for current injection and voltage detection, and attached along a chest circumference of a subject to be examined;
    a controller configured to selectively supply currents to at least one pair of electrodes selected from each of the plurality of first electrodes and the plurality of second electrodes, measure voltage through unselected electrodes, and generate an upper airway image and a thorax image based on upper airway impedance data and thorax impedance data separated from impedance data corresponding to the measured voltage; and
    an apnea diagnoser configured to diagnose apnea by quantifying the upper airway image and the thorax image.

2.  The apparatus for measuring the sleep apnea according to claim 1, wherein the controller comprises:

    a first current injection module configured to inject a current having a plurality of frequency ranges through at least one pair of electrodes selected among the plurality of first electrodes attached to the face and neck circumferences of the subject; and
    a second current injection module configured to inject a current having a plurality of frequency ranges through at least one pair of electrodes selected among the plurality of second electrodes attached to the chest circumference of the subject.

3.  The apparatus for measuring the sleep apnea according to claim 2, wherein one of the first current injection module and the second current injection module selects the selected pair of electrodes and a frequency, generates and converts a voltage signal based on the selected frequency into a current, and injects the converted currents to the face and neck and the thorax of the subject through the selected pairs of electrodes.

4.  The apparatus for measuring the sleep apnea according to claim 2, wherein the controller comprises:

    a voltage measuring module configured to measure voltages induced by the currents injected from the unselected electrodes among the plurality of first electrodes and the unselected electrodes among the plurality of second electrodes;
    an impedance data obtaining module configured to obtain impedance data based on the measured voltage;
    an algorithm function module configured to separate the upper airway impedance data and the thorax impedance data by applying a signal separating algorithm to the obtained impedance data, and restore the separated upper airway and thorax impedance data with regard to magnitudes; and
    an image generation module configured to generate the upper airway image and the thorax image based on the restored upper airway and thorax impedance data.

5.  The apparatus for measuring the sleep apnea according to claim 4, wherein:

    the voltage measuring module removes noises included in the detected voltage based on a slope of the measured voltage, and replaces a voltage corresponding to a section, a level of which is greater than a threshold, by a preset voltage when the slope of the detected voltage is higher than the threshold, and
    the impedance data obtaining module obtains the impedance data based on the voltage from which the noises are removed.

6.  The apparatus for measuring the sleep apnea according to claim 5, wherein the algorithm function module separates the upper airway impedance data and the thorax impedance data different from each other by applying the signal separating algorithm, i.e. an independent component analyses (ICA) algorithm to the obtained impedance data, and restores each of the separated impedance data to have a magnitude of an original signal by applying a signal magnitude restoration algorithm.

7. The apparatus for measuring the sleep apnea according to claim 4, wherein the controller further comprises a control module configured to control selection of at least one pair of electrodes from the plurality of first electrodes and the plurality of second electrodes, control selection of the unselected electrodes, and control sensing of a biometric signal from the subject.

8. The apparatus for measuring the sleep apnea according to claim 1, further comprising a sensing element configured to sense a biometric signal of the subject.

9. The apparatus for measuring the sleep apnea according to claim 8, wherein the apnea diagnoser is configured to quantify at least one of opening and obstruction change, degree and pattern of the upper airway over time based on the upper airway image, and quantify at least one of air distribution change, degree and pattern inside a lung over time based on the thorax image.

10. The apparatus for measuring the sleep apnea according to claim 9, wherein the apnea diagnoser is configured to diagnose at least one of obstructive apnea, central apnea, and complex apnea based on results from quantification of the upper airway image and the thorax image and the biometric signal sensed by the sensing element.

11. The apparatus for measuring the sleep apnea according to claim 1, wherein the plurality of first electrodes and the plurality of second electrodes comprise at least one of a simple electrode and a complex electrode, and are arrayed on one side of a base plate made of a flexible elastic material and attached to the upper airway and the thorax.

12. A method of measuring sleep apnea of a subject by an apparatus for measuring the sleep apnea, the method comprising:

   selectively supplying currents to at least one pair of electrodes selected from each of a plurality of first electrodes attached along face and neck circumferences of the subject and a plurality of second electrodes attached along a chest circumference of the subject;
   measuring voltage through unselected electrodes among the plurality of first electrodes and the plurality of second electrodes;
   obtaining impedance data based on the measured voltage;
   separating upper airway impedance data and thorax impedance data, which are different from each other, by applying a signal separating algorithm to the obtained impedance data;
   generating an upper airway image and a thorax image based on the upper airway impedance data and the thorax impedance data; and
   diagnosing apnea by quantifying the upper airway image and the thorax image.

13. The method according to claim 12, wherein the selective supply of the current comprises selecting a pair of electrodes selected among the plurality of first electrodes, a pair of electrodes selected among the plurality of second electrodes, and a frequency; generating and converting a voltage signal based on the selected frequency into a current; and injecting the converted currents to the face and neck and the thorax of the subject through the selected pairs of electrodes.

14. The method according to claim 12, wherein the separation of the data comprises separating the upper airway impedance data and the thorax impedance data different from each other by applying the signal separating algorithm, i.e. an independent component analyses (ICA) algorithm to the obtained impedance data; and restoring each of the separated impedance data to have a magnitude of an original signal by applying a signal magnitude restoration algorithm.

15. The method according to claim 14, wherein the generating of the image comprises generating the upper airway image and the thorax image from the restored upper airway and thorax impedance data.

16. The method according to claim 12, further comprising sensing a biometric signal of the subject.

17. The method according to claim 16, wherein the diagnosis of the apnea comprises diagnosing at least one of obstructive apnea, central apnea, and complex apnea based on results from quantification of the upper airway image and the thorax image and the sensed biometric signal.

18. A computer program stored in a computer readable recording medium to perform the method according to any one of claims 12 to 17.

Fig.1

100

130

110
UPPER AIRWAY
ELECTRODE ELEMENT

131
FIRST CURRENT
INJECTION MODULE

CONTROLLER 137

136
IMAGE GENERATION
MODULE

150
APNEA DIAGNOSER

CONTROL
MODULE

135
ALGORITHM
FUNCTION MODULE

134
IMPEDANCE DATA
OBTAINING MODULE

120
THORAX ELECTRODE
ELEMENT

132
SECOND CURRENT
INJECTION MODULE

133
VOLTAGE
MEASURING MODULE

SENSING ELEMENT

140

Fig.2a

110
20a
30a

140

120

20b

30b

Fig2b.

61a    61

+

40                                    30

20(22)

⇒

=

40                                    30

61a

⇒

61

Fig3a.

20

24

22

21

23

23    21    23    22

Fig.3b.

## 20'

22'

21'

Fig4a.

30

32

32

31

Fig.4b

Fig4c

Fig4d

SENSING ELEMENT

Fig4e

Fig5a

Fig.5b

INDEPENDENT SIGNAL : x[t]
(1xN)

ORIGINAL SIGNAL : y[t]
(208xN)

SIGNAL MAGNITUDE
RESTORATION
at[t] + b

INDEPENDENT SIGNAL WITH
RESTORED MAGNITUDE : x̄[t]
(208xN)

Fig6a

Fig.6b

Fig.7a

Fig.7b

Expiration                    Inspiration

Fig.8a

Fig.8b.

Expiration       Inspiration

Fig.9

```
                        ┌─────────┐
                        │  START  │
                        └────┬────┘
                             ▼
   ┌──────────────────────────────────────────┐
   │   SELECTIVELY SUPPLY CURRENTS TO PAIRS OF │──910
   │   ELECTRODES SELECTED FROM EACH OF PLURAL │
   │ FIRST ELECTRODES AND PLURAL SECOND ELECTRODES│
   └─────────────────────┬────────────────────┘
                         ▼
   ┌──────────────────────────────────────────┐
   │ MEASURE VOLTAGE THROUGH UNSELECTED ELECTRODES │──920
   └─────────────────────┬────────────────────┘
                         ▼
   ┌──────────────────────────────────────────┐
   │          OBTAIN IMPEDANCE DATA            │──930
   └─────────────────────┬────────────────────┘
                         ▼
   ┌──────────────────────────────────────────┐
   │     SEPARATE UPPER AIRWAY IMPEDANCE DATA  │──940
   │         AND THORAX IMPEDANCE DATA         │
   └─────────────────────┬────────────────────┘
                         ▼
   ┌──────────────────────────────────────────┐
   │  GENERATE UPPER AIRWAY IMAGE AND THORAX IMAGE │──950
   └─────────────────────┬────────────────────┘
                         ▼
   ┌──────────────────────────────────────────┐
   │       DIAGNOSE APNEA BY QUANTIFYING       │──960
   │    UPPER AIRWAY IMAGE AND THORAX IMAGE     │
   └─────────────────────┬────────────────────┘
                         ▼
                    ┌─────────┐
                    │   END   │
                    └─────────┘
```

## INTERNATIONAL SEARCH REPORT

| | International application No. |
|---|---|
| | **PCT/KR2017/012918** |

| A. | CLASSIFICATION OF SUBJECT MATTER |
|---|---|

*A61B 5/00(2006.01)i, A61B 5/053(2006.01)i*

According to International Patent Classification (IPC) or to both national classification and IPC

| B. | FIELDS SEARCHED |
|---|---|

Minimum documentation searched (classification system followed by classification symbols)
A61B 5/00; A61B 5/05; A61B 5/04; A61B 5/08; A61B 5/053; A61B 5/11; A61B 5/0452; A61B 5/0402

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: sleep, apnea, upper airway, breast, image

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2015-0123186 A (INDUSTRY-ACADEMIC COOPERATION FOUNDATION GYEONGSANG NATIONAL UNIVERSITY) 03 November 2015 See paragraphs [32]-[54]; claim 1; and figure 2. | 1-11,18 |
| Y | US 2010-0228143 A1 (TESCHNER et al.) 09 September 2010 See paragraphs [35]-[41]; claim 1; and figures 8, 9. | 1-11,18 |
| Y | KR 10-0700112 B1 (UNIVERSITY-INDUSTRY COOPERATION GROUP OF KYUNG HEE UNIVERSITY et al.) 28 March 2007 See paragraphs [31]-[35]; claims 3, 4; and figure 1. | 3,5,6 |
| Y | JP 2008-504073 A (CARDIAC PACEMAKERS INC.) 14 February 2008 See paragraphs [10]-[12], [55]; and claim 1. | 4-10 |
| A | KR 10-2015-0033197 A (SAMSUNG ELECTRONICS CO., LTD. et al.) 01 April 2015 See paragraphs [32]-[55]; claim 1; and figures 1, 2. | 1-11,18 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 FEBRUARY 2018 (12.02.2018) | **13 FEBRUARY 2018 (13.02.2018)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

## INTERNATIONAL SEARCH REPORT

International application No.

**PCT/KR2017/012918**

| Box No. II | Observations where certain claims were found unsearchable (Continuation of item 2 of first sheet) |
| --- | --- |

This international search report has not been established in respect of certain claims under Article 17(2)(a) for the following reasons:

1. ☒ Claims Nos.: **12-17**
   because they relate to subject matter not required to be searched by this Authority, namely:

   Claims 12-17 pertain to a method for treatment of the human body by surgery or therapy, and to a diagnostic method, and thus pertain to subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
| --- | --- |

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT
Information on patent family members

International application No.

**PCT/KR2017/012918**

| Patent document cited in search report | Publication date | Patent family member | Publication date |
|---|---|---|---|
| KR 10-2015-0123186 A | 03/11/2015 | EP 3135192 A1<br>KR 10-1649680 B1<br>US 2017-0079544 A1<br>WO 2015-163710 A1 | 01/03/2017<br>23/08/2016<br>23/03/2017<br>29/10/2015 |
| US 2010-0228143 A1 | 09/09/2010 | EP 2228009 A1<br>US 8321007 B2 | 15/09/2010<br>27/11/2012 |
| KR 10-0700112 B1 | 28/03/2007 | EP 1978870 A1<br>EP 1978870 A4<br>US 2008-0252304 A1<br>US 7847565 B2<br>WO 2007-089062 A1 | 15/10/2008<br>09/09/2015<br>16/10/2008<br>07/12/2010<br>09/08/2007 |
| JP 2008-504073 A | 14/02/2008 | EP 1773188 A1<br>JP 4819805 B2<br>US 2005-0288600 A1<br>US 2010-0198283 A1<br>US 7706866 B2<br>US 7941206 B2<br>WO 2006-002398 A1 | 18/04/2007<br>24/11/2011<br>29/12/2005<br>05/08/2010<br>27/04/2010<br>10/05/2011<br>05/01/2006 |
| KR 10-2015-0033197 A | 01/04/2015 | US 2015-0087930 A1 | 26/03/2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PANOSSIAN LA ; AVIDAN AY.** Review of sleep disorders. *Med Clin North Am,* 2009, vol. 93 (2), 407-25
- **WOODSON BT ; FRANCO R.** Physiology of sleep disordered breathing. *Otolaryngol Clin North Am,* 2007, vol. 40 (4), 691-711
- **COLEMAN J.** Overview of sleep disorders: where does obstructive sleep apnea syndrome fit. *Otolaryngol Clin North Am,* 1999, vol. 32 (2), 187-93
- **REDLINE S ; STROHL KP.** Recognition and consequences of obstructive sleep apnea hyponea syndrome. *Otolaryngol Clin North Am,* 1999, vol. 32 (2), 303-31
- **THAKKAR K ; YAO M.** Diagnostic studies in obstructive sleep apnea. *Otolaryngol Clin North Am,* 2007, vol. 40 (4), 785-805